# EUROPEAN PATENT SPECIFICATION

(11) **EP 2 477 548 B1**
(45) Date of publication and mention of the grant of the patent: **26.12.2012**
(21) Application number: 10760061.1
(22) Date of filing: 08.09.2010
(51) Int. Cl.: A61B 6/12

(54) **DEPTH DISAMBIGUATION OF INTERVENTIONAL INSTRUMENTS FROM A SINGLE X-RAY PROJECTION IMAGE AND ITS CALIBRATION**
TIEFENUNTERSCHEIDUNG VON EINGRIFFSINSTRUMENTEN AUS EINEM EINZELNEN RÖNTGENPROJEKTIONSBILD UND SEINER KALIBRIERUNG
DÉSAMBIGUÏSATION DE PROFONDEUR D'INSTRUMENTS D'INTERVENTION À PARTIR D'UNE IMAGE DE PROJECTION PAR RAYONS X UNIQUE ET DE L'ÉTALONNAGE DE CELLE-CI

(30) Priority: 15.09.2009 EP 09305849
(43) Date of publication of application: 25.07.2012
(73) Proprietor: Koninklijke Philips Electronics N.V., 5621 BA Eindhoven (NL)
(72) Inventor: CATHIER, Pascal, NL-5656 AE Eindhoven (NL); GOGIN, Nicolaas Pierre Bruno, NL-5656 AE Eindhoven (NL); FLORENT, Raoul, NL-5656 AE Eindhoven (NL)
(74) Representative: Van Velzen, Maaike Mathilde
(86) International application number: PCT/IB2010/054046
(87) International publication number: WO 2011/033419

(56) References cited:
- DE-A1-102008 006 516
- US-A1- 2006 241 413
- US-A1- 2006 285 638
- US-A1- 2008 043 902
- US-A1- 2008 208 040

## Description

### FIELD OF THE INVENTION

The present invention relates to x-ray guided procedures. Especially, the invention relates to a method for determining a depth of an instrument in an object. Furthermore, the invention relates to a computer program for determining a depth of an instrument in an object as well as to an x-ray device equipped with such a computer program.

### BACKGROUND OF THE INVENTION

Electrophysiology is a specific domain of interventional cardiology where physicians use intracardiac catheters to locate and cure electrical dysfunctions of the heart rhythm, under x-ray fluoroscopy guidance. A very challenging electrophysiology procedure is radio-frequency ablation for the treatment of an atrial fibrillation, also called AF.

Electrophysiologics need a special training to perfectly know the anatomy and the excess pathways to all the sites of interest and some practice to select the correct devices and manipulate them to target. The patient's anatomy can be recorded with 3D-imaging devices (CT, MRI) or by injecting contrast agent locally just at the beginning of the intervention (left atrium (LA) and ostium of the pulmonary veins (PV) for AF and coronary veins and sinus for cardiac resynchronization therapy (CRT)), but the physician has to perform mental registration to navigate in the life fluoro images where this information is not visible any more.

For AF procedures, knowing the exact position of the catheters when measuring electrical potentials is a key to found the sources that cause fibrillation (ectopic foci, reentry loop). Even more important is anatomical mapping of the ablation sites in order to perform the desired ablation pattern (such as PV isolation or roof line ablation in LA).

Document US-A1-2006/241413 discloses a method for determining a depth of an instrument in an object comprising the steps of generating one x-ray projection image of the instrument in the object and discriminating on the basis of a segmentation of the object between possible locations of the tip of the instrument in the object.

### SUMMARY OF THE INVENTION

X-ray images are projective, meaning that the 3D geometry is flattened along projection lines going from the source to the detector. In particular procedures, such as mapping or ablation, the interventional instrument lies on the wall of the organ. Using a 3D segmentation of this organ registered to the x-ray, the instrument necessarily lies on the intersection of this surface with its projection line. The line and the surface typically intersect with a segmentation surface at a discrete number of points (typically 2 for shapes such as the anterior of the LA). One then has just to disambiguate between these different possible locations to determine the exact location of the instrument. In this invention, it is proposed to use the apparent width of the instrument measured in x-ray images to accomplish this task.

It is an object of the invention to provide a method for determining a depth of an instrument in an object, wherein the object is exposed by radiation as less as possible.

It is a further object of the invention to provide a computer program such that the method according to the invention may be substantially automated.

This is achieved by the subject matter of the respective independent claims. Further embodiments are described in the respective dependent claims.

In general, this is achieved by a method for determining a depth of an instrument in an object, the method comprising the steps of generating one x-ray projection image of the instrument in the object, estimating the size of a portion of the instrument in the object, and discriminating between possible locations of the portion of the instrument in the object, on the basis of the estimated size and of a segmentation of the object.

According to another embodiment of the invention, the step of estimating of the size includes either width estimation, a 2D geometric model, or a 3D geometric model.

Therefore, a more robust use of the width estimation is made, by using it to discriminate between possible locations using a segmentation of the anatomy, rather than estimating the absolute depth of the instrument. Furthermore, a calibration process makes it robust to possible errors in the projection matrix.

When an anatomical model of the volume of intervention is available and registered with a projection image (e.g. x-ray-CT registration), knowing the 3D position of an interventional tool allows positioning it within the model, thus facilitating navigation and site mapping.

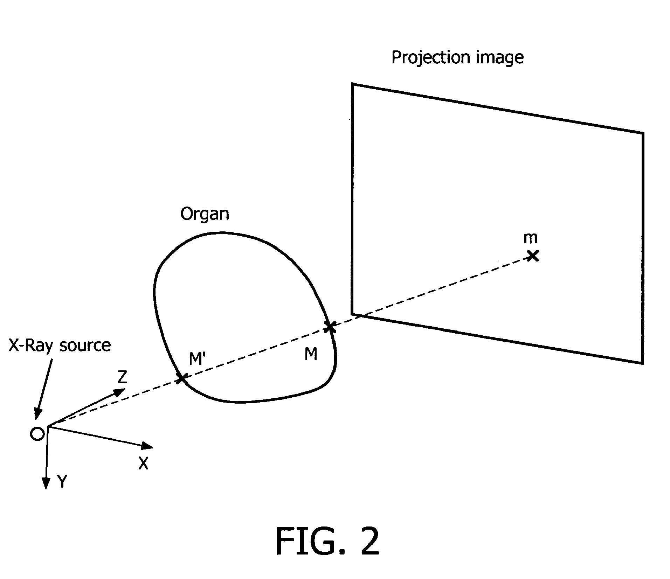

According to the method according to the invention, the 3D position of an interventional tool from a single 2D projection is estimated using constraints based on the corrected size of the interventional tool, a prior segmentation of the organ within which the interventional tool lies registered to the x-ray image, and the knowledge that the interventional tool is pressed against one of the wall of this organ, which is the case during some interventional procedures such as atrial fibrillation of mapping. Under these assumptions, a projection m of the interventional tool corresponds to only a few possible 3D locations, e.g. M and M' (in figure 2). The measurement of the apparent size of the interventional tool is used to discriminate between these possible locations.

According to another embodiment of the invention, the step of discriminating of the method according to the invention is further based on the information as to whether the instrument is placed at an anterior or posterior position, which means, is based on the information as to whether the instrument is pushed forward or pulled back inside the organ.

According to yet another embodiment of the invention the method further comprises the step of calibrating the estimation of the size by the way of estimating the size at the anterior and the posterior positions.

This may include computing of size thresholds. Having such thresholds may provide for the further advantage that also any kind of slight movements of the object or relative movements of inner parts of said object may not affect the intended discrimination. In case that the object is a living body, such movements may be caused by breathing or a heartbeat.

Accordingly, the first part of the invention relates to the estimation of the apparent size of the instrument. The apparent size relates only to the tip of the instrument, which is assumed to be in contact with the organ. The size of the tip is estimated using either a 2D or 3D geometric model if its shape is known beforehand or using non-specific techniques such as a width estimator, which requires only a limited set of geometric assumptions (e.g. constant width at the tip).

The second part of the invention relates to the calibration of such measurements. To ensure proper calibration of sizes with respect to possible locations, a calibration step is needed during which the operator is asked to place the instrument at an anterior than at a posterior position. At these known locations, the system records the estimated sizes and uses this data to compute optimal size thresholds used to discriminate between these positions. The system can also decide from the data that the precision is not high enough to discriminate between positions and take appropriate steps, such as warning the user and disabling the automatic depth disambiguation feature.

Accordingly, a major aspect of the invention is that it may not be necessary to have absolute size measurements. A certain inaccuracy may be tolerated since the discrimination between two possible positions may also be reliable on the basis of such inaccurate measurements.

It is noted that the instrument might be, on the one hand, a flexible or stiff catheter, and on the other hand also a biopsy device, a cannula or trocar.

According to another aspect of the invention, a computer program is provided, by means of which the above described method may be performed automatically, or at least predominantly automatically. Therefore, the computer program comprises sets of instructions for storing a projection image generated by the x-ray system, sets of instructions for identifying and estimating the size of a portion of an instrument shown in the projection image, and sets of instructions for discriminating between possible locations of the portion of the instrument, based on the estimated size as well as on a segmentation of the object.

Further, the computer program may comprise sets of instruction for loading data from a data base including previously recorded image information, or may comprise sets of instructions for information retrieval from a user.

According to a further aspect of the invention, an x-ray device or system is proposed, which comprises an x-ray source, an x-ray detector, and a processing unit for controlling the x-ray source and the x-ray detector, wherein the processing unit further includes the above-mentioned computer program which may be stored in the processing unit.

Such a computer program is preferably loaded into a work memory of a data processor. The data processor is thus equipped to carry out the method of the invention. Further, the invention relates to a computer readable medium, such as a CD-ROM, at which the computer program may be stored. However, the computer program may also be presented over a network like the World Wide Web and can be downloaded into the work memory of a data processor form such a network.

It has to be noted that embodiments of the invention are described with reference to different subject matters. In particular, some embodiments are described with reference to method type claims whereas other embodiments are described with reference to apparatus type claims. However, a person skilled in the art will gather from the above and the following description that, unless other notified, in addition to any combination of features belonging to one type of subject matter also any combination between features relating to different subject matters is considered to be disclosed with this application.

The aspects defined above and further aspects, features and advantages of the present invention can also be derived from the examples of the embodiments to be described herein after and are explained with reference to examples of embodiments also shown in the figures, but to which the invention is not limited.

### BRIEF DESCRIPTION OF THE DRAWINGS

Figure 1 shows a flow chart of the method according to the invention.
Figure 2 shows an illustration of how a projection image may be achieved.
Figure 3 shows an example of a projection image.
Figure 4 shows an example of a system according to the invention.

### DETAILED DESCRIPTION OF EMBODIMENTS

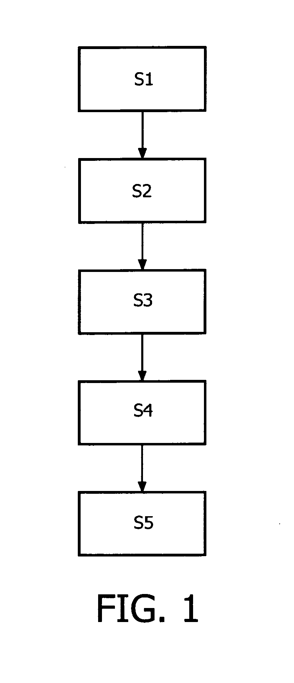

Figure 1 is a flow chart, showing the steps of a method for determining a depth of an instrument in an object according to the invention. It will be understood that the steps described with respect to the method are major steps, wherein these major steps might be differentiated or divided into several sub steps. Furthermore, there might be also sub steps between these major steps. Therefore, a sub step is only mentioned if that step is important for the understanding of the principles of the method according to the invention.

In step S1 of the method according to the invention one x-ray projection image of the instrument in the object is generated. To reduce the radiation to which for example a patient is exposed, only a single projection image is generated.

In step S2, the size of a portion of the instrument in the object is estimated. Usually, this portion will be the tip portion of an instrument, i.e. the portion of a catheter at which for example ablation electrodes are provided.

The projected width of the catheter is measured by optimizing a goodness-of fit measure between the image and a width-measuring kernel. The kernel is composed of two parts, each designed to fit on one side of the catheter. Each part is composed of a weight function, and an edge model. The weight function is a smooth kernel, elongated in the direction of the catheter axis (typically an anisotropic Gaussian kernel). Both parts of the kernel are constrained to be parallel to one another. The edge function is a monotonically increasing function modeling a step edge (typically a linear function). The kernel is thus parameterized by the position of its center, its rotation angle, and its width. The goodness-of-fit function is the sum of the two weighted correlation between each edge function and the image; the weights are given by the associated weight function. The function is optimized with respect to the parameters of the kernel, in a multiscale fashion. The kernel width at the optimum yields our measure of the projected width.

In step S3, it is discriminated between possible locations of the portion of the instrument in the object. Having in mind that there may be only a few possible locations, in case the instrument is introduced into a specific anatomical structure, it has only to be decided as to whether the questionable portion of the instrument is more or less deeper inside the object.

To improve the results of the method of the invention, in step S4, the estimation of the size is calibrated by way of estimating the size at an anterior position and at a posterior position. Therefore, a system may ask a user to first pull the instrument such that the tip portion of the instrument may contact the wall inside the organ at an anterior position and then may ask the user to push the instrument into a posterior position with contact to an opposite wall of the inner lumen of the organ.

A sub step of step S4 may be that size thresholds are computed on the basis of these estimations at the anterior and posterior positions. Such thresholds may help in further estimations of the size to discriminate the position of the instrument.

As a final step S5, the system may output information which may help the user to decide at which location the questionable portion of the instrument is currently.

Figure 2 is a schematically illustration of how a projection image may be generated. An x-ray image gives a 2D projection of a 3D volume. As can be seen in figure 2, a radiation beam starting at the x-ray source will enter into an organ at a point M' (an anterior position), will pass through the organ to an exist point M (a posterior position), and will subsequently impinge the two-dimensional plane or surface of a detector at point m. Though it is not possible to retrieve depth information of a 3D object simply from a single projection point, this task becomes possible when additionally knowledge on the geometrical structure of the object such as orientations, distances, and angles is used.

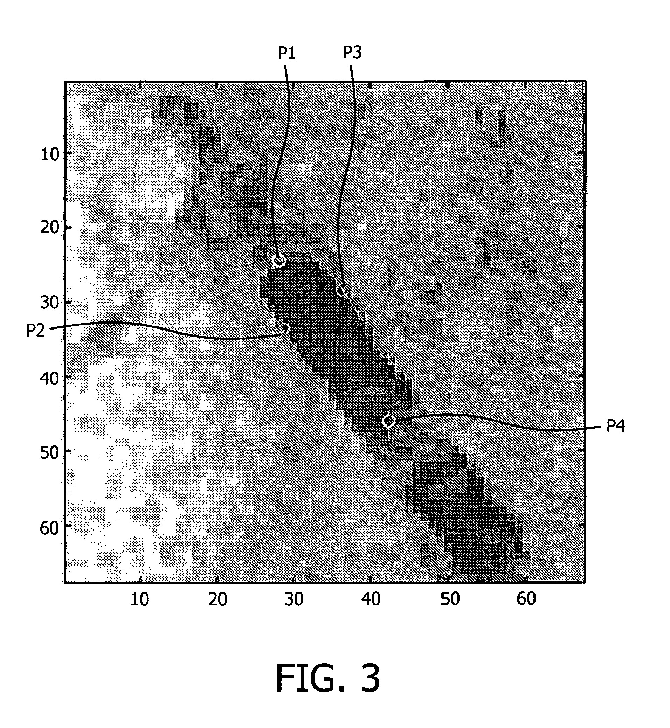

Figure 3 shows an example of apparent size estimation using the width of the projected instrument tip. The numbers at the vertical left as well as the bottom horizontal edge of said example are for scale. The additionally shown small circles, indicated as P1 to P4, define edge points of the tip portions of the instrument into substantially perpendicular to each other orientated directions. These circles or points may be used as a basis for the estimation for the size of the portion of the instrument.

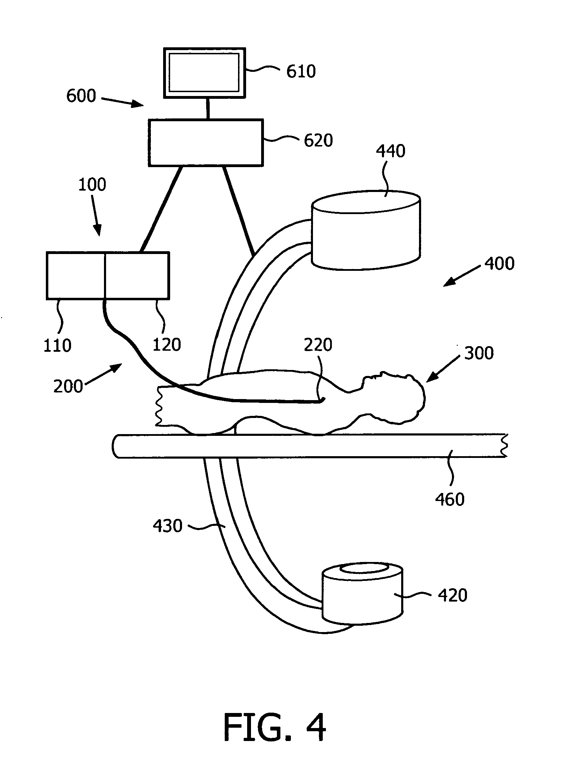

Figure 4 shows an exemplary system according to the invention, the system including a console 100 for an instrument 200, which instrument may be introduced into a patient 300. Furthermore, an x-ray device 400 is arranged relative to the patient such that a projection image of a region may be generated in which the tip portion of the catheter 200 is located. Finally, a processing unit 600 is provided which may control the generating of the projection image by means of the x-ray device 400, as well as the console 100 to control functions of the catheter 200, if appropriate.

Here, the controller 100 may include a unit for providing electrical pulses or signals for an electrical ablation of tissue in the organ. On the other hand, the console 100 may comprise a further device 120 by means of which for example the orientation of the tip portion 220 of the catheter 200 may be controlled, or by means of which drugs or a contrast agent may be delivered.

The x-ray device 400 includes an x-ray source 420 as well as a detector for x-ray radiation 440, wherein both, the x-ray source 420 as well as the x-ray detector 440 are arranged at a C-arm 430 to ensure a proper orientation of both, relative to each other. The patient 300 may be positioned at a table 460.

The processing unit 600 includes first of all a control unit 620 and further a monitor 610, wherein an output of information with respect to the discriminated depth may be shown on said monitor.

While the invention has been illustrated and described in detail in the drawings and afore-going description, such illustrations and descriptions are to be considered illustrative or exemplary and not restrictive, the invention is not limited to the disclosed embodiments.

Other variations to the disclosed embodiments can be understood and effected by those skilled in the art in practicing the claimed invention, from a study of the drawings, the disclosure and the appended claims. In the claims, the word comprising does not exclude other elements or steps, and the indefinite article a or an does not exclude a plurality. A single processor or other unit may fulfill the functions of several items recited in the claims. The mere fact that certain measures are recited and mutually different dependent claims does not indicate that a combination of these measured cannot be used to advantage. A computer program may be stored/distributed on a suitable medium such as an optical storage medium or a solid-state medium supplied together with or as a part of another hardware, but may also be distributed in other forms, such as via the internet or other wired or wireless telecommunication systems. Any reference signs in the claims should not be construed as limiting the scope.

### LIST OF REFERENCE SIGNS:

- 100: console
- 110: device for electrical ablation
- 120: device for delivering a fluid
- 200: catheter
- 220: tip of catheter
- 300: patient
- 400: x-ray device
- 420: x-ray source
- 430: C-arm
- 440: x-ray detector
- 460: table
- 600: processing unit
- 610: monitor
- 620: control device

## Claims

1. A method for determining a depth of an instrument in an object, the method comprising the steps of
generating one x-ray projection image of the instrument in the object,
estimating the size of a portion of the instrument (220) in the object (300),
discriminating on the basis of the estimated size and on the basis of a segmentation of the object, between possible locations of the portion of the instrument in the object.

2. The method of claim 1, wherein the step of estimating of the size includes width estimation.

3. The method of claim 1, wherein the step of estimating of the size includes a 2D or 3D geometric model.

4. The method of claim 1, wherein the step of discriminating is further based on the information as to whether the instrument is placed at an anterior or a posterior position.

5. The method of claim 4, the method further comprising the step of calibrating the estimation of the size by the way of estimating the size at the anterior position and at the posterior position.

6. The method of claim 5, wherein the step of calibrating includes computing size thresholds.

7. A computer program for determining a depth of an instrument in an object, the computer program comprising sets of instructions for performing the method according to claim 1.

8. An x-ray device comprising
a x-ray source (420),
a x-ray detector (440),
a processing unit (600) for controlling the x-ray source and the x-ray detector, and
a computer program for determining a depth of an instrument in an object according to claim 7, stored in the processing unit.

## Patentansprüche

1. Verfahren zur Bestimmung einer Tiefe eines Instruments in einem Objekt, wobei das Verfahren die folgenden Schritte umfasst:
Erzeugen eines Röntgenprojektionsbildes des Instruments in dem Objekt,
Schätzen der Größe eines Teils des Instruments (220) in dem Objekt (300),
Unterscheiden zwischen möglichen Positionen des Teils des Instruments in dem Objekt ausgehend von der geschätzten Größe und ausgehend von einer Segmentierung des Objekts.

2. Verfahren nach Anspruch 1, wobei der Schritt des Schätzens der Größe eine Breitenschätzung umfasst.

3. Verfahren nach Anspruch 1, wobei der Schritt des Schätzens der Größe ein geometrisches 2D- oder 3D-Modell umfasst.

4. Verfahren nach Anspruch 1, wobei der Schritt des Unterscheidens weiterhin auf der Information basiert, ob das Instrument an einer anterioren oder einer posterioren Position platziert ist.

5. Verfahren nach Anspruch 4, wobei das Verfahren weiterhin den Schritt des Kalibrierens der Größenschätzung mittels Schätzen der Größe an der anterioren Position und der posterioren Position umfasst.

6. Verfahren nach Anspruch 5, wobei der Schritt des Kalibrierens das Berechnen von Größen-Schwellenwerten umfasst.

7. Computerprogramm zur Bestimmung einer Tiefe eines Instruments in einem Objekt, wobei das Computerprogramm Anweisungssätze zur Durchführung des Verfahrens nach Anspruch 1 umfasst.

8. Röntgenvorrichtung mit
einer Röntgenquelle (420),
einem Röntgendetektor (440),
einer Verarbeitungseinheit (600) zur Steuerung der Röntgenquelle und des Röntgendetektors und
einem Computerprogramm zur Bestimmung einer Tiefe eines Instruments in einem Objekt nach Anspruch 7, das in der Verarbeitungseinheit gespeichert ist.

## Revendications

1. Procédé de détermination d'une profondeur d'un instrument dans un objet, le procédé comprenant les étapes de :
la génération d'une image de projection par rayons X de l'instrument dans l'objet,
l'estimation de la taille d'une portion de l'instrument (220) dans l'objet (300),
la discrimination sur la base de la taille estimée et sur la base d'une segmentation de l'objet, entre des emplacements possibles de la portion de l'instrument dans l'objet.

2. Procédé selon la revendication 1, dans lequel l'étape de l'estimation de la taille comprend l'estimation de la largeur.

3. Procédé selon la revendication 1, dans lequel l'étape de l'estimation de la taille comprend un modèle géométrique 2D ou 3D.

4. Procédé selon la revendication 1, dans lequel l'étape de la discrimination est en outre basée sur les informations indiquant si l'instrument est placé à une position antérieure ou postérieure.

5. Procédé selon la revendication 4, le procédé comprenant en outre l'étape de l'étalonnage de l'estimation de la taille par le biais de l'estimation de la taille à la position antérieure et à la position postérieure.

6. Procédé selon la revendication 5, dans lequel l'étape de l'étalonnage comprend le calcul de seuils de taille.

7. Programme informatique pour déterminer une profondeur d'un instrument dans un objet, le programme informatique comprenant des ensembles d'instructions pour effectuer le procédé selon la revendication 1.

8. Dispositif à rayons X comprenant :
une source de rayons X (420),
un détecteur de rayons X (440),
une unité de traitement (600) pour commander la source de rayons X et le détecteur de rayons X, et
un programme informatique pour déterminer une profondeur d'un instrument dans un objet selon la revendication 7, stocké dans l'unité de traitement.
